# EUROPÄISCHE PATENTSCHRIFT

(11) **EP 3 295 906 B1**
(45) Veröffentlichungstag und Bekanntmachung des Hinweises auf die Patenterteilung: **28.07.2021**
(21) Anmeldenummer: 16188792.2
(22) Anmeldetag: 14.09.2016
(51) Int. Cl.: A61F 9/007, A61M 1/00

(54) **SYSTEM ZUM DURCHFÜHREN EINER PHAKOEMULSIFIKATION**
SYSTEM FOR PERFORMING A PHACOEMULSIFICATION
SYSTÈME D'EXÉCUTION D'UNE PHACOÉMULSIFICATION

(43) Veröffentlichungstag der Anmeldung: 21.03.2018
(73) Patentinhaber: Fritz Ruck Ophthalmologische Systeme GmbH, 52249 Eschweiler (DE)
(72) Erfinder: Kerkhoff, Franciscus Theodorus, 5581TK Waalre (NL); Klomp, Manfred, 6336 AM Hulsberg (NL)
(74) Vertreter: Schwarz & Partner Patentanwälte OG

(56) Entgegenhaltungen:
- EP-A1- 2 674 176
- US-A- 5 342 293
- US-A1- 2011 092 896

## Beschreibung

Die Erfindung betrifft ein System zum Durchführen einer Phakoemulsifikation, umfassend ein Handstück mit einem Werkzeug gebildet durch eine Hohlnadel und einer die Hohlnadel umgebenden Mantelfläche, wobei die Hohlnadel einen ersten Kanal ausbildet und zwischen Hohlnadel und Mantelfläche ein im Querschnitt kreisringförmiger zweiter Kanal ausgebildet ist, eine Irrigationsvorrichtung, welche zur Abgabe eines Fluids mit einem Druck ausgebildet ist, eine Aspirationsvorrichtung, welche zum Ansaugen des Fluids ausgebildet ist, eine Verteilervorrichtung, wobei die Verteilervorrichtung mit dem ersten Kanal, mit dem zweiten Kanal, der Irrigationsvorrichtung und der Aspirationsvorrichtung zum Austausch von Fluiden verbunden ist, und eine Steuereinrichtung, welche mit der Irrigationsvorrichtung, der Aspirationsvorrichtung und der Verteilervorrichtung zum Austausch von Informationen verbunden ist, wobei die Verteilervorrichtung in einem ersten Betriebsmodus den zweiten Kanal und die Irrigationsvorrichtung zum Austausch von Fluiden verbindet und den ersten Kanal und die Aspirationsvorrichtung zum Austausch von Fluiden verbindet und wobei die Verteilervorrichtung in einem zweiten Betriebsmodus den ersten Kanal und die Irrigationsvorrichtung zum Austausch von Fluiden verbindet.

Der Stand der Technik wird durch die Dokumente US2011/092896 und US5342293 gebildet. US2011/092896 offenbart ein Operationssystem zur Durchführung einer Phakoemulsifikation, bei dem eine Verstopfung des Aspirationskanals durch Zuleitung von Flüssigkeit in den Aspirationskanal gelöst werden kann. Dokument US5342293 offenbart ein Phakoemulsifikationssystem, das die Flüssigkeitszufuhr, d.h. den Druck in der Irrigationsleitung linear steuern kann.

Zum Durchführen einer Phakoemulsifikation am Auge wird das Auge zuerst durch einen Schnitt in der Hornhaut geöffnet. Der Schnitt muss dabei so groß ausgebildet sein, dass eine neue Linse aufgerollt ins Auge eingeführt werden kann. Durch den Schnitt wird mittels einer Nadel ein Kapselsack des Auges kreisförmig geöffnet, in dem die Linse des Auges sitzt. Um die Linse vom Kapselsack zu lösen wird in weiterer Folge eine sehr dünne Kanüle in den Schnitt eingeführt und zwischen Kapselsack und Linse geschoben. Die Kanüle ist an eine Spritze angeschlossen, mittels welcher ein Operateur Fluid zum Lösen der Linse zwischen die Linse und den Kapselsack spritzen kann. Überflüssiges Fluid fließt durch den Schnitt an einem Schaft der Kanüle vorbei aus dem Auge, wobei aufgrund der dünnen Kanüle und aufgrund der Größe des Schnitts ein Spalt zwischen Kanüle und Schnitt sehr groß ist. Anschließend daran wird ein Werkzeug angebracht an einem Handstück zur Durchführung der Phakoemulsifikation durch den Schnitt ins Auge eingeführt. Das Werkzeug ist durch eine Hohlnadel und einer die Hohlnadel umgebenden Mantelfläche gebildet, wobei die Hohlnadel einen ersten Kanal ausbildet und zwischen Hohlnadel und Mantelfläche ein zweiter im Querschnitt kreisringförmiger zweiter Kanal ausgebildet ist. Die Hohlnadel wird zum Schwingen im Ultraschallbereich angeregt, wodurch sich die Linse in kleine Teile zertrümmern lässt, wobei die abgespalteten Teile der Linse mittels der Aspirationsvorrichtung durch den ersten Kanal aus dem Auge abgesaugt werden. Während der Operation wird dem Auge konstant über den zweiten Kanal Irrigationsfluid zugeführt, um einen Innendruck im Auge möglichst konstant zu halten. Ein Druck des dem Auge zugeführten Irrigationsfluids wird mittels der Irrigationsvorrichtung und einer Steuereinrichtung stufenweise gesteuert. So ein System zum Steuern des Drucks ist zum Beispiel aus der Patentanmeldung EP 2 674 176 A1 bekannt. Die Regelung des Innendrucks ist immens wichtig, da es bei einem zu kleinen Innendruck, sowie bei einem zu großen Innendruck zu irreparablen Schäden im Auge kommen kann. Zur Vermeidung, dass während dem Absaugen der in Teile zerbrochenen Linse anderes Gewebe durch den ersten Kanal angesaugt wird, kann kurzzeitig durch den ersten Kanal rückgespült werden, wodurch irrtümlich angesaugtes Gewebe ausgestoßen wird.

Beim Lösen der Linse vom Kapselsack mittels der Kanüle und Spritze hat sich als nachteilig erwiesen, dass der Operateur mit der Spritze aufgrund einer inneren Reibung der Spritze die Menge an Fluid und damit einen Fluiddruck, mit dem das Fluid in das Auge abgegeben wird, schlecht kontrollieren kann. Wie bereits beschrieben ist ein zu großer Innendruck im Auge unbedingt zu vermeiden. Ferner hat sich als nachteilig erwiesen, dass es aufgrund der Größe des Spalts zwischen Kanüle und Schnitt durch das aus dem Auge fließende Fluid am Schaft der Kanüle vorbei zu einem partiellen Ausschwemmen einer Iris aus dem Auge kommen kann. Dies tritt vor allem bei Personen auf, die Alfa-Blocker ähnliche Medikamente zu sich nehmen, da diese Medikamente zur Erschlaffung der Iris führen.

Es ist die Aufgabe der vorliegenden Erfindung ein System zum Durchführen einer Phakoemulsifikation bereitzustellen, welches zum Durchführen von weiteren Eingriffen am Auge eines Patienten herangezogen werden kann.

Die Aufgabe wird erfindungsgemäß dadurch gelöst, dass im zweiten Betriebsmodus mittels der Steuereinrichtung der Druck linear steuerbar ist, mit dem die Irrigationsvorrichtung Fluid an die Verteilervorrichtung abgibt.

Durch die erfindungsgemäße Ausbildung des Systems zum Durchführen einer Phakoemulsifikation können mittels des Systems auch andere Eingriffe an Augen eines Patienten durchgeführt werden. Durch den linear steuerbaren Druck, mit dem die Irrigationsvorrichtung Fluid im zweiten Betriebsmodus an die Verteilvorrichtung abgibt, kann vor der Durchführung der Phakoemulsifikation mit dem erfindungsgemäßen System auch die Linse vom Kapselsack gelöst werden. Somit kann auf die Verwendung der Kanüle und der Spritze zum Ablösen der Linse vom Kapselsack verzichtet werden, wodurch ein Wechsel zwischen den Operationsgeräten vermieden wird und der operative Eingriff schneller vollzogen werden kann. Darüber hinaus ist bei der Nutzung des erfindungsgemäßen Systems der Vorteil erhalten, dass das Werkzeug, das in das Auge eingeführt wird, am Schaft einen großen Durchmesser aufweist. Hierdurch wird der in seiner Größe im Wesentlichen definierte Schnitt in der Hornhaut besser ausgefüllt, wodurch sich zwischen Werkzeug und Schnitt nur ein geringer Spalt ergibt und das Ausschwemmen der Iris verhindert wird.

Zweckmäßig ist die Irrigationsvorrichtung durch einen Behälter gebildet, der mittels einer Pumpe gesteuert durch die Steuereinrichtung durch eine Zufuhr von Gas in den Behälter mit einem Überdruck beaufschlagbar ist, wodurch der Druck, mit dem die Irrigationsvorrichtung Fluid an die Verteilervorrichtung abgibt, gesteuert wird. Vorteilhaft weist die Steuereinrichtung einen durch ein Fußpedal gebildeten Fußschalter auf, wobei die Steuereinrichtung im ersten Betriebsmodus und/oder zweiten Betriebsmodus den Überdruck und somit den Druck, mit dem die Irrigationsvorrichtung Fluid an die Verteilervorrichtung abgibt, proportional zu einer Winkelstellung des Fußpedals steuert. Hierdurch ist das erfindungsgemäße System für einen Operateur sehr einfach zu handhaben und der Druck ist für den Operateur sehr gut dosierbar.

In einer weiteren Ausführungsvariante ist die Irrigationsvorrichtung durch einen Behälter gebildet, welcher mittels einer Hebevorrichtung gesteuert durch die Steuereinrichtung in vertikaler Richtung verlagerbar ist, wodurch der Druck, mit dem Fluid aus dem Behälter an die Verteilervorrichtung abgeben wird, verändert wird. In diesem Zusammenhang ist auch vorteilhaft, wenn die Steuereinrichtung einen durch ein Fußpedal gebildeten Fußschalter aufweist, wobei die Steuereinrichtung im ersten Betriebsmodus und/oder zweiten Betriebsmodus eine Lage des Behälters in vertikaler Richtung proportional zur Winkelstellung des Fußpedals steuert.

Zweckmäßig weist die Steuereinrichtung ferner einen Schalter auf, welcher entweder am Fußschalter oder an dem Handstück angebracht ist und zum Umschalten der Betriebsmodi ausgebildet ist. Hierdurch ist der Vorteil erhalten, dass der Operateur, ohne das Handstück aus der Hand geben zu müssen, zwischen den Betriebsmodi hin und her wechseln kann.

Bevorzugt ist die Aspirationsvorrichtung durch eine mit Vakuum beaufschlagte Kassette oder durch eine Peristaltikpumpe gebildet, die zum Absaugen des Fluids adaptiert ist, wobei ein Unterdruck, mit dem Fluid abgesaugt wird über die Steuereinrichtung steuerbar ist.

Weitere vorteilhafte Ausführungsvarianten des erfindungsgemäßen Systems werden in weiterer Folge anhand der Figuren näher erläutert.
Figur 1 zeigt eine Ausführungsvariante eines erfindungsgemäßen Systems in einer schematischen Ansicht.
Figur 2 zeigt eine Ausführungsvariante einer Verteilervorrichtung des erfindungsgemäßen Systems nach Figur 1 in einer schematischen Ansicht.
Figuren 3 bis 5 zeigen eine Ausführungsvariante eines Werkzeugs des erfindungsgemäßen Systems nach Figur 1 in einer schematischen Ansicht während einem Eingriff am Auge eines Patienten bei unterschiedlichem Operationsfortschritt.

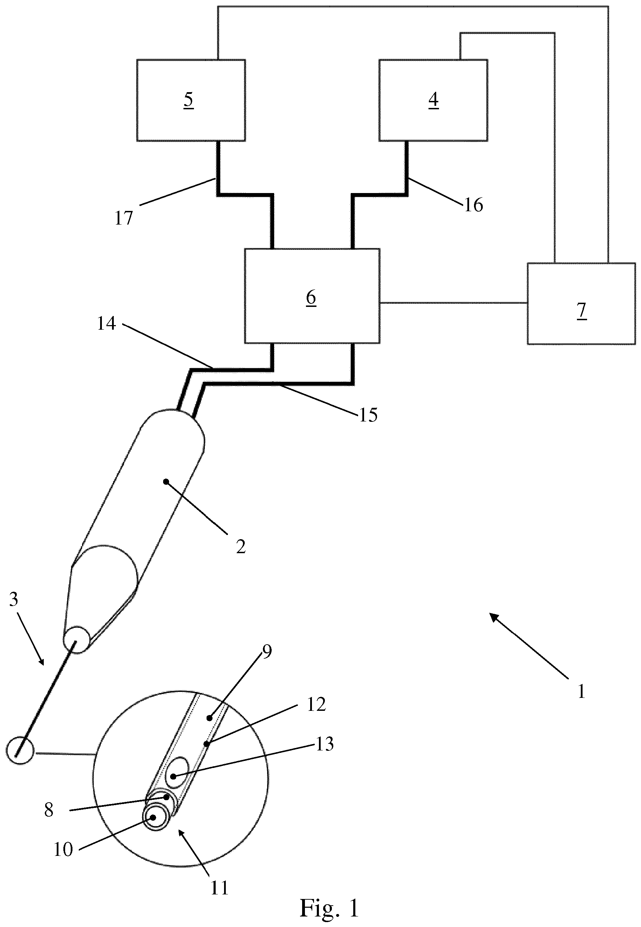

Figur 1 zeigt eine Ausführungsvariante eines erfindungsgemäßen Systems 1 zum Durchführen einer Phakoemulsifikation in einer schematischen Ansicht. Das erfindungsgemäße System 1 umfasst ein Handstück 2 mit einem Werkzeug 3, eine Irrigationsvorrichtung 4, eine Aspirationsvorrichtung 5, eine Verteilervorrichtung 6 und eine Steuereinrichtung 7. Das Werkzeug 3 ist durch eine Hohlnadel 8 und eine die Hohlnadel 8 umgebende Mantelfläche 9 gebildet. Die Hohlnadel 8 bildet einen ersten Kanal 10 aus, welcher an einem offenen Ende 11 der Hohlnadel 8 mündet, und ist vorteilhaft aus Edelstahl gebildet. Die Mantelfläche 9 ist vorteilhaft durch ein Rohr aus Metall oder Kunststoff, oder durch einen Kunststoffschlauch gebildet, welches Rohr bzw. welcher Schlauch im Bereich des offenen Endes 11 an der Hohlnadel 8 dichtend abschließt. Zwischen der Hohlnadel 8 und der Mantelfläche 9 ist ein zweiter im Querschnitt kreisringförmiger Kanal 12 ausgebildet, welcher in zwei einander gegenüberliegende Öffnungen 13 mündet. Die Verteilervorrichtung 6 ist mittels einer ersten Leitung 14 mit dem ersten Kanal 10 des Werkzeugs 3, mittels einer zweiten Leitung 15 mit dem zweiten Kanal 12 des Werkzeugs 3, mittels einer dritten Leitung 16 mit der Irrigationsvorrichtung 4 und mittels einer vierten Leitung 17 mit der Aspirationsvorrichtung 5 zum Austausch von Fluiden verbunden. Die Steuereinrichtung 7 ist zum Austausch von Informationen mit der Irrigationsvorrichtung 4, mit der Aspirationsvorrichtung 5 und mit der Verteilervorrichtung 6 verbunden und ist vorteilhaft durch einen Mikrocontroller oder einen Computer gebildet.

Die Irrigationsvorrichtung 4 ist durch einen Behälter, welcher ein Fluid, insbesondere Irrigationsfluid, beinhaltet, und durch eine Pumpe gebildet, welche zur Zufuhr von einem gasförmigen Medium, insbesondere Luft, in den Behälter zur Erzeugung eines Überdrucks im Behälter ausgebildet ist. Die Aspirationsvorrichtung 5 ist durch eine Peristaltikpumpe gebildet, welche zum Ansaugen des Fluids konfiguriert ist. Sowohl die Gaszufuhr in den Behälter der Irrigationsvorrichtung 4, als auch ein Unterdruck, mit dem Fluid durch die Peristaltikpumpe angesaugt wird, wird durch die Steuereinrichtung 7 gesteuert. Vorteilhaft weist die Steuereinrichtung 7 zum Steuern der Gaszufuhr in den Behälter und somit zum Steuern eines Drucks, mit dem Fluid aus dem Behälter abgegeben wird, einen durch ein Fußpedal gebildeten Fußschalter auf, wobei der Druck, mit dem Fluid aus dem Behälter abgegeben wird proportional zu einer Winkelstellung des Fußpedals ist.

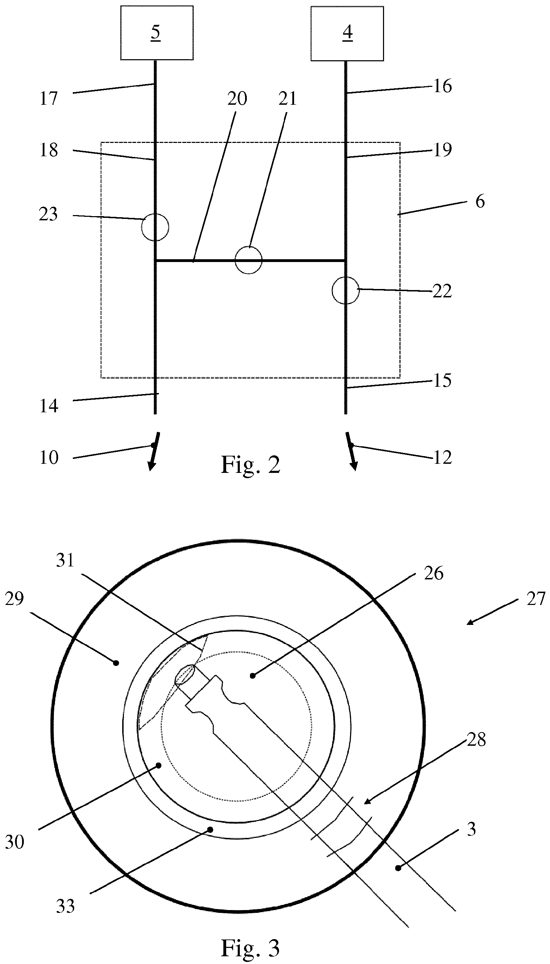

Figur 2 zeigt eine Ausführungsvariante einer Verteilervorrichtung 6 des erfindungsgemäßen Systems 1 nach Figur 1 in einer schematischen Ansicht. Die Verteilervorrichtung 6 umfasst eine die Aspirationsvorrichtung 5 und den ersten Kanal 10 verbindende Aspirationsleitung 18, eine die Irrigationsvorrichtung 4 und den zweiten Kanal 12 verbindende Irrigationsleitung 19, eine die Aspirationsleitung 18 und die Irrigationsleitung 19 verbindende Verbindungsleitung 20 sowie drei Ventile. Ein erstes Ventil 21 ist in der Verbindungsleitung 20 ausgebildet. Ein zweites Ventil 22 ist zwischen einem Bereich, in dem die Verbindungsleitung 20 in die Irrigationsleitung 19 mündet, und der zweiten Leitung 15 ausgebildet, welche zweite Leitung 15 in den zweiten Kanal 12 mündet. Ein drittes Ventil 23 ist zwischen einem Bereich, in dem die Verbindungsleitung 20 in die Aspirationsleitung 18 mündet, und der vierten Leitung 17 ausgebildet, welche vierte Leitung 17 in die Aspirationsvorrichtung 5 mündet. Der erste Kanal 10 und der zweite Kanal 12 sind in Figur 2 durch jeweilige Pfeile 10 und 12 stilisiert. Die Ventile der Verteilervorrichtung 6 werden durch die Steuereinrichtung 7 angesteuert.

In einer weiteren Ausführungsvariante weist die Verteilvorrichtung 6 eine Umgehungsleitung auf, welche an die Irrigationsleitung 19 zwischen dem zweiten Ventil 22 und der zweiten Leitung 15, die in den zweiten Kanal 12 mündet, anschließt und in die Aspirationsleitung 18 zwischen dem dritten Ventil 23 und der vierten Leitung 17 mündet. In der Umgehungsleitung ist ein viertes Ventil ausgebildet.

Figuren 3 bis 5 zeigen eine Ausführungsvariante eines Werkzeugs 3 des erfindungsgemäßen Systems 1 nach Figur 1 in einer schematischen Ansicht während einem Eingriff am Auge 27 eines Patienten bei unterschiedlichem Operationsfortschritt. Das Auge 27 des Patienten wurde bereits durch einen Schnitt 28 in einer Hornhaut 29 des Auges 27 geöffnet und es wurde bereits ein Kapselsack 30 des Auges 27 mittels einer Nadel im Wesentlichen kreisförmig geöffnet.

Im Folgenden wird eine Verwendung des erfindungsgemäßen Systems 1 zum Ablösen einer Linse 26 vom Kapselsack 30 eines Auges 27 und zum Durchführen einer anschließenden Phakoemulsifikation näher beschrieben. Das erfindungsgemäße System 1 kann in zwei Betriebsmodi betrieben werden, wobei im ersten Betriebsmodus das erste Ventil 21 geschlossen ist und das zweite Ventil 22 und das dritte Ventil 23 der Verteilervorrichtung 6 geöffnet sind. Infolge ist die Irrigationsvorrichtung 4 mit dem zweiten Kanal 12 zum Austausch von Fluiden verbunden und die Aspirationsvorrichtung 5 mit dem ersten Kanal 10 zum Austausch von Fluiden verbunden. Ferner wird im ersten Betriebsmodus das Werkzeug 3 mittels eines in dem Handstück 2 ausgebildeten nicht dargestellten Antriebsmittels in eine mechanische Schwingung versetzt. In einem zweiten Betriebsmodus ist das zweite Ventil 22 und das dritte Ventil 23 geschlossen und das erste Ventil 21 geöffnet. Infolge ist die Irrigationsvorrichtung 4 mit dem ersten Kanal 10 zum Austausch von Fluiden verbunden.

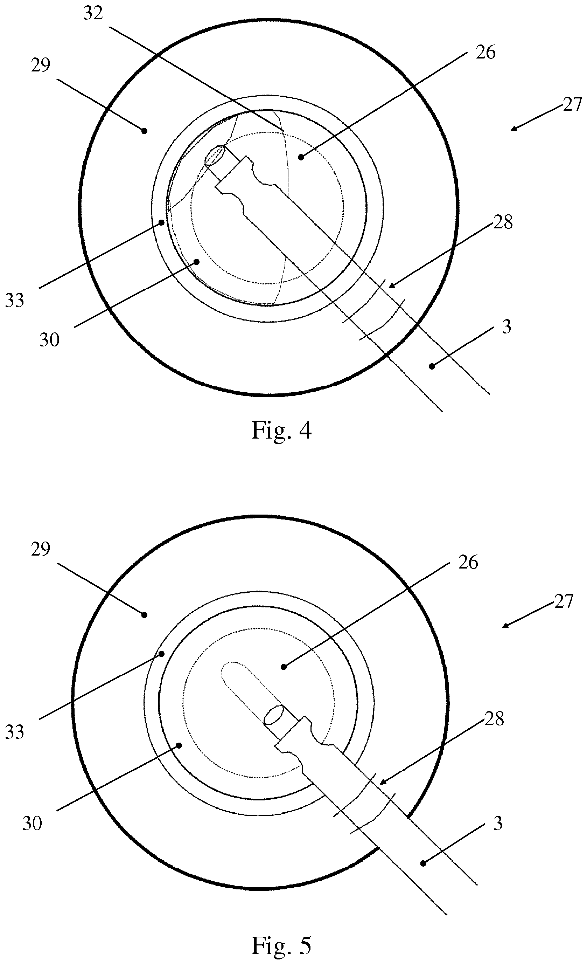

Zum Ablösen der Linse 26 von dem Kapselsack 30 stellt der Operateur an der Steuereinrichtung 7 den zweiten Betriebsmodus ein. Dazu weist die Steuereinrichtung 7 vorteilhaft einen Schalter auf, welcher zum Beispiel an dem Handstück 2 oder an dem Fußpedal ausgebildet ist. Zum Ablösen der Linse 26 wird die Hohlnadel 8 zwischen die Linse 26 und den Kapselsack 30 geschoben. Über das Fußpedal der Steuereinrichtung 7 kann der Operateur nun einen Druck, mit dem Fluid aus dem Behälter der Irrigationsvorrichtung 4 abgegeben wird, und infolge einen Austrittsdruck, mit dem Fluid aus dem ersten Kanal 10 aus dem Werkzeug 3 austritt, steuern, wobei die Steuereinrichtung 7 den Druck so steuert, dass dieser proportional zu einer Winkelstellung des Fußpedals 7 ist und ein Austrittsdruck am ersten Kanal 10 von ca. 120 mm WaterColumn nicht überschritten wird. Das Fluid wird durch den Druck zwischen die Linse 26 und den Kapselsack 30 gedrückt und breitet sich dazwischen aus, wodurch die Linse 26 von dem Kapselsack 30 getrennt wird. In Figur 3 und Figur 4 sind Fluidfronten 31 und 32 bei unterschiedlichem Ausbreitungsfortschritt des Fluids im Auge 27 abgebildet. Bei gelöster Linse 26 kann die Linse im Kapselsack 30 gedreht werden. Während dem Einspritzen von Fluid in das Auge 27 über den ersten Kanal 10 fließt überschüssiges Fluid durch einen Spalt zwischen einem Schaft des Werkzeugs 3 und dem Schnitt 28 aus dem Auge 27 ab, wobei der Spalt durch die Dicke des Werkzeugs 3 so klein ist, dass ein Ausschwemmen einer Iris 33 des Auges 27 aus dem Auge 27 verhindert wird. Weist die Verteilervorrichtung 6 einen Umgehungsleitung mit einem vierten Ventil auf, kann das überschüssiges Fluid auch durch Öffnen des vierten Ventils über die Aspirationsvorrichtung 5 über den zweiten Kanal 12 aus dem Auge 27 abgesaugt werden.

In weiterer Folge schaltet der Operateur mittels des Schalters der Steuereinrichtung 7 auf den ersten Betriebsmodus um, um die Phakoemulsifikation durchzuführen. Im ersten Betriebsmodus wird durch den zweiten Kanal 12 Fluid in das Auge 27 abgegeben und Fluid über den ersten Kanal 10 aus dem Auge 27 abgesaugt. Vorteilhaft wird im ersten Betriebsmodus ein Druck, mit dem Fluid aus der Irrigationsvorrichtung 4 abgegeben wird, nicht mehr linear geregelt, sondern stufenweise. Sobald das Fußpedal auch nur geringfügig vom Operateur betätigt wird, wird der Druck, mit dem Fluid aus der Irrigationsvorrichtung 4 abgegeben wird, auf einen fest vorher eingestellten Wert eingestellt. Der Druck, mit dem Fluid aus der Irrigationsvorrichtung 4 im ersten Betriebsmodus abgegeben wird, wird über eine separate Stelleinrichtung der Steuereinrichtung 7 eingestellt und justiert. Hierdurch ist der Vorteil erhalten, dass der Operateur sich auf den operativen Eingriff konzentrieren kann und nicht ständig über die Winkelstellung des Fußpedals den Druck korrigieren muss. Bei der Phakoemulsifikation wird entsprechend Figur 5 die Linse 26 in einzelne Teile zertrümmert, die über den ersten Kanal 10 abgesaugt werden.

Es sei hier noch erwähnt, dass die Verteilervorrichtung auch durch ein einfaches Wegeventil, insbesondere ein 4/2 Wegeventil, gebildet sein kann.

## Patentansprüche

1. System (1) zum Durchführen einer Phakoemulsifikation, umfassend ein Handstück (2) mit einem Werkzeug (3) gebildet durch eine Hohlnadel (8) und einer die Hohlnadel (8) umgebenden Mantelfläche (9), wobei die Hohlnadel (8) einen ersten Kanal (10) ausbildet und zwischen Hohlnadel (8) und Mantelfläche (9) ein im Querschnitt kreisringförmiger zweiter Kanal (12) ausgebildet ist,
eine Irrigationsvorrichtung (4), welche zur Abgabe des Fluids mit einem Druck ausgebildet ist, eine Aspirationsvorrichtung (5), welche zum Ansaugen des Fluids ausgebildet ist, eine Verteilervorrichtung (6), wobei die Verteilervorrichtung (6) mit dem ersten Kanal (10), mit dem zweiten Kanal (12), der Irrigationsvorrichtung (4) und der Aspirationsvorrichtung (5) zum Austausch von Fluiden verbunden ist, und eine Steuereinrichtung (7), welche mit der Irrigationsvorrichtung (4), der Aspirationsvorrichtung (5) und der Verteilervorrichtung (6) zum Austausch von Informationen verbunden ist,
wobei die Verteilervorrichtung (6) in einem ersten Betriebsmodus den zweiten Kanal (12) und die Irrigationsvorrichtung (4) zum Austausch von Fluiden verbindet und den ersten Kanal (10) und die Aspirationsvorrichtung (5) zum Austausch von Fluiden verbindet **dadurch gekennzeichnet dass** die Verteilervorrichtung (6) in einem zweiten Betriebsmodus den ersten Kanal (10) und die Irrigationsvorrichtung (4) zum Austausch von Fluiden verbindet, und im zweiten Betriebsmodus mittels der Steuereinrichtung (7) der Druck linear steuerbar ist, mit dem die Irrigationsvorrichtung (4) Fluid an die Verteilervorrichtung (6) abgibt.

2. System (1) nach Anspruch 1, **dadurch gekennzeichnet, dass** die Verteilervorrichtung (6) eine die Aspirationsvorrichtung (5) und den ersten Kanal (10) des Handstücks (2) verbindende Aspirationsleitung (18), eine die Irrigationsvorrichtung (4) und den zweiten Kanal (12) des Handstücks (2) verbindende Irrigationsleitung (19), eine die Aspirationsleitung (18) und die Irrigationsleitung (19) verbindende Verbindungsleitung (20) sowie drei Ventile aufweist, wobei in der Verbindungsleitung (20) ein erstes Ventil (21) ausgebildet ist, in der Irrigationsleitung (19) zwischen einem Bereich, in dem die Verbindungsleitung (20) in die Irrigationsleitung (19) mündet, und dem zweiten Kanal (12) ein zweites Ventil (22) ausgebildet ist und in der Aspirationsleitung (18) zwischen Aspirationsvorrichtung (5) und einem Bereich, in dem die Verbindungsleitung (20) in die Aspirationsleitung (18) mündet, ein drittes Ventil (23) ausgebildet ist und wobei im ersten Betriebsmodus das zweite Ventil (22) und das dritte Ventil (23) geöffnet sind und das erste Ventil (21) geschlossen ist und im zweiten Betriebsmodus das zweite Ventil (22) und das dritte Ventil (23) geschlossen sind und das erste Ventil (21) geöffnet ist.

3. System nach Anspruch 2, **dadurch gekennzeichnet, dass** die Verteilervorrichtung eine Umgehungsleitung aufweist, welche an die Irrigationsleitung zwischen dem zweiten Ventil und dem zweiten Kanal anschließt und in die Aspirationsleitung zwischen dem dritten Ventil und der Aspirationsvorrichtung mündet, wobei in der Umgehungsleitung ein viertes Ventil ausgebildet ist, welches im ersten Betriebsmodus geschlossen ist und im zweiten Betriebsmodus offen ist.

4. System (1) nach einem der vorherigen Ansprüche, **dadurch gekennzeichnet**, das die Steuereinrichtung (7) zum Steuern des Drucks einen Fußschalter aufweist und zum Umschalten zwischen den Betriebsmodi einen per Hand oder Fuß betätigbaren Schalter aufweist.

5. System (1) nach einem der vorgehenden Ansprüche, **dadurch gekennzeichnet, dass** die Irrigationsvorrichtung (4) durch einen Behälter gebildet ist, der mittels einer Pumpe gesteuert durch die Steuereinrichtung (7) mit einem Überdruck beaufschlagbar ist, und/oder dass die Irrigationsvorrichtung (4) durch einen Behälter gebildet ist, welcher mittels einer Hebevorrichtung gesteuert durch die Steuereinrichtung (7) in vertikaler Richtung verlagerbar ist.

6. System (1) nach einem der vorgehenden Ansprüche, **dadurch gekennzeichnet, dass** die Aspirationsvorrichtung (5) durch eine mit Vakuum beaufschlagte Kassette oder durch eine Peristaltikpumpe gebildet ist, welche Peristaltikpumpe zum Ansaugen von Fluid adaptiert ist.

## Claims

1. A system (1) for performing a phacoemulsification, comprising a tool holder (2) having a tool (3) formed by a hollow needle (8) and a shell surface (9) surrounding the hollow needle (8), wherein the hollow needle (8) forms a first a channel (10) and wherein there is formed between hollow needle (8) and shell surface (9) a second channel (12) having a circular cross-section,
an irrigation device (4), which is configured to discharge the fluid with a pressure, an aspiration device (5), which is configured to aspire the fluid, a distributor device (6), wherein the distributor device (6) is connected to the first channel (10), to the second channel (12), to the irrigation device (4) and the aspiration device (5) for the exchange of fluids, and a control device (7), which is connected to the irrigation device (4), the aspiration device (5) and the distributor device (6) for the exchange of information,
wherein the distributor device (6) connects in a first operational mode the second channel (12) and the irrigation device (4) for the exchange of fluids and the first channel (10) and the aspiration device (5) for the exchange of fluids, **characterized in that** the distributor device (6) connects in a second operational mode the first channel (10) and the irrigation device (4) for the exchange of fluids and that the pressure is controllable in the second operational mode in a linear way by means of the control device (7), at which pressure the irrigation device (4) discharges the fluid to the distributor device (6).

2. A system (1) according to claim 1, **characterized in that** the distributor device (6) has an aspiration line (18) connecting the aspiration device (5) and the first channel (10) of the tool holder (2), an irrigation line (19) connecting the irrigation device (4) and the second channel (12) of the tool holder (2), a connection line (20) connecting the aspiration line (18) and the irrigation line (19) as well as three valves, wherein in the connection line (20) there is formed a first valve (21), in the irrigation line (19) between a region, in which the connection line (20) ends into the irrigation line (19), and the second channel (12) there is formed a second valve (22) and in the aspiration line (18) between the aspiration device (5) and a region, in which the connection line (20) ends into the aspiration line (18), there is formed a third valve (23), and wherein in the first operational mode the second valve (22) and the third valve (23) are opened and the first valve is closed and wherein in the second operational mode the second valve (22) and the third valve (23) are closed and the first valve (21) is opened.

3. A system according to claim 2, **characterized in that** the distributor device has a bypass line, which connects to the irrigation line between the second valve and the second channel and ends into the aspiration line between the third valve and the aspiration device, wherein in the bypass line there is formed a fourth valve, which is closed in the first operational mode and open in the second operational mode.

4. A system (1) according to any of the preceding claims, **characterized in that** the control device (7) has a foot switch for controlling the pressure and a switch that may be actuated by hand or foot for switching between the operational modes.

5. A system (1) according to any of the preceding claims, **characterized in that** the irrigation device (4) is formed by a container which may be pressurized to an excess pressure by means of a pump that is controlled by the control device (7) and/or that the irrigation device (4) is formed by a container, which may be shifted in a vertical direction by means of a lifting device that is controlled by the control device (7).

6. A system (1) according to any of the preceding claims, **characterized in that** the aspiration device (5) is formed by a cartridge under vacuum or by a peristaltic pump, which peristaltic pump is adapted to aspire fluid.

## Revendications

1. Système (1) pour effectuer une phacoémulsification, comprenant :
une pièce à main (2) présentant un outil (3) formé par une aiguille creuse (8) et une surface d'enveloppe (9) qui entoure l'aiguille creuse (8), dans lequel l'aiguille creuse (8) forme un premier canal (10) et un second canal (12) de section transversale annulaire est formé entre l'aiguille creuse (8) et la surface d'enveloppe (9), et
un dispositif d'irrigation (4) qui prévu pour la délivrance du fluide avec une pression, un dispositif d'aspiration (5) qui est prévu pour l'aspiration du fluide et, un dispositif de distribution (6), dans lequel le dispositif de distribution (6) est relié au premier canal (10), au second canal (12), au dispositif d'irrigation (4) ainsi qu'au dispositif d'aspiration (5) pour l'échange de fluides, et un appareil de commande (7) qui est relié au dispositif d'irrigation (4), au dispositif d'aspiration (5) ainsi qu'au dispositif de distribution (6) pour l'échange d'informations,
dans lequel, dans un premier mode de fonctionnement, le dispositif de distribution (6) relie le second canal (12) au dispositif d'irrigation (4) pour l'échange de fluides et relie le premier canal (10) au dispositif d'aspiration (5) pour l'échange de fluides,
**caractérisé en ce que**, dans un second mode de fonctionnement, le dispositif de distribution (6) relie le premier canal (10) au dispositif d'irrigation (4) pour l'échange de fluides et, dans le second mode de fonctionnement, la pression avec laquelle le dispositif d'irrigation (4) délivre le fluide au dispositif de distribution (6) peut être commandée de façon linéaire à l'aide de l'appareil de commande (7).

2. Système (1) selon la revendication 1, **caractérisé en ce que** le dispositif de distribution (6) comprend une conduite d'aspiration (18) qui relie le dispositif d'aspiration (5) au premier canal (10) de la pièce à main (2), une conduite d'irrigation (19) qui relie le dispositif d'irrigation (4) au second canal (12) de la pièce à main (2), une conduite de liaison (20) qui relie la conduite d'aspiration (18) à la conduite d'irrigation (19) ainsi que trois soupapes, dans lequel une première soupape (21) est réalisée à l'intérieur de la conduite de liaison (20), une deuxième soupape (22) est réalisée à l'intérieur de la conduite d'irrigation (19) entre une zone dans laquelle la conduite de liaison (20) débouche à l'intérieur de la conduite d'irrigation (19) et le second canal (12) et une troisième soupape (23) est réalisée à l'intérieur de la conduite d'aspiration (18) entre le dispositif d'aspiration (5) et une zone dans laquelle la conduite de liaison (20) débouche à l'intérieur de la conduite d'aspiration (18), et dans lequel, dans le premier mode de fonctionnement, la deuxième soupape (22) et la troisième soupape (23) sont ouvertes et la première soupape (21) est fermée et, dans le second mode de fonctionnement, la deuxième soupape (22) et la troisième soupape (23) sont fermées et la première soupape (21) est ouverte.

3. Système selon la revendication 2, **caractérisé en ce que** le dispositif de distribution comprend une conduite de dérivation qui est raccordée à la conduite d'irrigation entre la deuxième soupape et le second canal et qui débouche à l'intérieur de la conduite d'aspiration entre la troisième soupape et le dispositif d'aspiration, dans lequel une quatrième soupape est réalisée à l'intérieur de la conduite de dérivation et est fermée dans le premier mode de fonctionnement et est ouverte dans le second mode de fonctionnement.

4. Système (1) selon l'une quelconque des revendications précédentes, **caractérisé en ce que** l'appareil de commande (7) comprend un interrupteur actionnable au pied pour la commande de la pression et un interrupteur actionnable à la main ou au pied pour le basculement entre les modes de fonctionnement.

5. Système (1) selon l'une quelconque des revendications précédentes, **caractérisé en ce que** le dispositif d'irrigation (4) est formé par un contenant qui peut être sollicité avec une surpression par l'appareil de commande (7) de façon commandée à l'aide d'une pompe, et/ou **en ce que** le dispositif d'irrigation (4) est formé par un contenant qui peut être déplacé dans la direction verticale de façon commandée par l'appareil de commande (7) à l'aide d'un dispositif de levage.

6. Système (1) selon l'une quelconque des revendications précédentes, **caractérisé en ce que** le dispositif d'aspiration (5) est formé par une cassette sollicitée avec un vide ou par une pompe péristaltique, ladite pompe péristaltique étant adaptée à l'aspiration de fluide.
